# EUROPEAN PATENT APPLICATION

(11) **EP 4 254 428 A1**
(43) Date of publication of application: **04.10.2023**
(21) Application number: 22176045.7
(22) Date of filing: 30.05.2022
(51) Int. Cl.: G16H 50/50, G16H 20/40, G16H 30/40, G16H 50/20, A61B 8/08, A61M 25/10

(54) **INTRAVASCULAR PROCEDURE STEP PREDICTION**

(30) Priority: 28.03.2022 US 202263324257 P
(71) Applicant: Koninklijke Philips N.V., 5656 AG Eindhoven (NL)
(72) Inventor: SALEHI, Leili, Eindhoven (NL); TOPOREK, Grzegorz Andrzej, Eindhoven (NL); SINHA, Ayushi, 5656AG Eindhoven (NL); ERKAMP, Ramon Quido, 5656AG Eindhoven (NL); PANSE, Ashish Sattyavrat, Eindhoven (NL)
(74) Representative: Philips Intellectual Property & Standards

(57) **Abstract**

A computer-implemented method of recommending a subsequent step in the performance of an intravascular procedure on a vessel, is provided. The method includes: inputting into a neural network one or more plaque characteristic maps for the vessel, and 2D angiographic data comprising a temporal sequence of images representing the vessel during the intravascular procedure; predicting, using the neural network, a value of a success metric obtained by performing one or more candidate intravascular procedure steps on the vessel with one or more candidate intravascular devices; and outputting one of the candidate intravascular procedure steps as the recommended subsequent step based on the predicted value of the one or more success metrics.

## Description

### TECHNICAL FIELD

The present disclosure relates to recommending a subsequent step in the performance of an intravascular procedure on a vessel. A computer-implemented method, a computer program product, and a system, are disclosed.

### BACKGROUND

Atherosclerosis is a leading cause of global mortality and morbidity. According to the WHO report "Global Health Estimates 2016: Deaths by Cause, Age, Sex, by Country and by Region, 2000-2016", Geneva, World Health Organization, 2018, cardiovascular diseases, principally atherosclerosis, are responsible for approximately 30% of world deaths. Atherosclerosis is an underlying cause of cardiovascular disease "CVD" in which plaques that are made up of fat, cholesterol, calcium and other substances build up in the walls of arteries. These plaques cause the arteries to harden and narrow, restricting the blood flow and supply of oxygen to vital organs, increasing the risk of blood clots that could potentially block the flow of blood to the heart or brain. Such narrowings are also referred-to as stenoses. Over time, the plaque may open, or rupture, forming a thrombus, or blood clot, further restricting the blood flow. The thrombus may also break away as an embolus. The embolus can become lodged elsewhere in the body and form an embolism that likewise blocks the artery. Thromboses may occur in various parts of the body, including in the heart and the brain, where their effects can be severe unless treated quickly. In the brain, for example, a thrombus, or an embolism, can lead to conditions such as (ischemic) stroke.

Various imaging techniques have been used to assess vascular plaque. The goal of such techniques is often to determine whether the plaque is "high risk", or in other words "vulnerable", i.e. that it has a high likelihood of causing thrombotic complications, such as myocardial infarction or stroke. This information is used by physicians to determine the course of treatment of the plaque.

Intravascular ultrasound "IVUS" imaging is typically considered to be the gold standard for assessing vascular plaque. However, IVUS is currently used in less than 10% of vascular catheterization operations. This is due to various limitations, including its size, cost, and the steep learning curve to image interpretation. The use of IVUS is also reported to increase the procedure time by about 34%, as mentioned in a document by Mariani, J. et. al., "Intravascular Ultrasound Guidance to Minimize the use of Iodine Contrast in Percutaneous Coronary Intervention: The MOZART Randomized Controlled Trial", JACC Cardiovascular Interventions, Volume 7, Issue 11, November 2014, Pages 1287 -93.

Extra-corporeal imaging techniques have also been used to assess intravascular plaques. For instance, a document by Conte, E. et. al., "Plaque quantification by coronary computed tomography angiography using intravascular ultrasound as a reference standard: a comparison between standard and last generation computed tomography scanners", European Heart Journal - Cardiovascular Imaging, Volume 21, Issue 2, February 2020, Pages 191-201, has demonstrated a good correlation between the results of the quantification of atherosclerotic plaque based on the images acquired by the latest generation of computed tomography angiography "CTA" scanners and IVUS systems. Based on their results, last generation CTA may be used for accurate non-invasive assessment and quantification of coronary plaques.

Morphological features of the stenosis such as the degree of lumen reduction, positive vessel wall remodeling, and the plaque location have also been used as markers of plaque rupture risk, as detailed in a document by Tomaniak, M. et. al., "Vulnerable plaques and patients: state-of-the-art", European Heart Journal, Volume 41, Issue 31, 14 August 2020, Pages 2997- 3004. Recent studies have demonstrated automatic characterization of the plaque using pathologic and morphological properties of plaque extracted from computed tomography angiography "CTA", or magnetic resonance "MR" images, and neural networks, as disclosed in a document by Hampe, N. et. al., "Machine Learning for Assessment of Coronary Artery Disease in Cardiac CT: A Survey, Frontiers in Cardiovascular Medicine", Volume 6, Article 172, 26 November 2019; and in a document by Cano-Espinosa, C. et. al., "Automated Agatston Score Computation in non-ECG Gated CT Scans Using Deep Learning", Proceedings of SPIE International Society of Optical Engineering, 2 March 2018, 10574; and in a document by Zreik, M. et. al., "A Recurrent CNN for Automatic Detection and Classification of Coronary Artery Plaque and Stenosis in Coronary CT Angiography", IEEE transaction of medical imaging, Volume 38, issue 7, July 2019, pages 1588-98; and in the document by Motwani, M. et. al., "Machine learning for prediction of all-cause mortality in patients with suspected coronary artery disease: a 5-year multicenter prospective registry analysis", European Heart Journal, Volume 38, Issue 7, 14 February 2017, Pages 500- 507. However, the accuracy of these morphological approaches typically does not exceed 80%.

Other studies have shown that plaque material properties, and structural stress are related to its vulnerability. For example, a document by Sadat, U. et. al., "Biomechanical structural stresses of atherosclerotic plaques, Expert Review of Cardiovascular Therapy", Volume 8, Issue 10, October 2010, Pages 1469 - 81, describes the extraction of this information from MR images and suggested the biomechanical features to be also considered to overcome the shortcomings of the morphology-based approaches in identifying patients at risk.

As outlined in the above discussion, various imaging techniques are known for use in determining the pathological, morphological, biomechanical, and material properties of a plaque in a vessel. The properties of the plaque are typically described by plaque characteristic maps, i.e. spatial distributions of these properties. Various techniques are also known for assessing these plaque characteristic maps, and for determining whether the plaque represented in the plaque characteristic maps is "high risk", or in other words "vulnerable", i.e. that it has a high likelihood of causing thrombotic complications, such as myocardial infarction or stroke.

Physicians use such risk information to decide on the course of treatment of plaque in a vessel. For example, physicians use this information to decide on the immediacy with which to treat a vessel, or which procedural steps to take in the procedure, i.e. whether to perform a balloon angioplasty procedure to widen an obstructed vessel, whether to also insert a stent, whether to perform a mechanical thrombectomy procedure, i.e. a mechanical removal of the thrombus, and so forth. Physicians also use this information do decide which type of treatment device to use. For example, the physician may use this information to decide which type of stent to use, or whether to extract the thrombus using a stent retriever device, or a direct aspiration device. These choices can have a significant impact on the outcome of the procedure. The risk information helps a physician to determine the course of treatment. However, many of the procedural steps are chosen based to the experience of the physician, which results in variable procedure outcomes.

Thus, there remains a need to provide improved support to a physician in order to help guide the physician in deciding which procedural steps to take during an intravascular procedure on a vessel.

### SUMMARY

According to one aspect of the present disclosure, a computer-implemented method of recommending a subsequent step in the performance of an intravascular procedure on a vessel, is provided. The method includes:
receiving one or more plaque characteristic maps for the vessel;
receiving 2D angiographic data comprising a temporal sequence of images representing the vessel during the intravascular procedure;
inputting the one or more plaque characteristic maps, and the 2D angiographic data, into a neural network;
predicting, using the neural network, a value of a success metric obtained by performing one or more candidate intravascular procedure steps on the vessel with one or more candidate intravascular devices; and
outputting one of the candidate intravascular procedure steps as the recommended subsequent step based on the predicted value of the one or more success metrics.

Since, in the above method, the outputted recommended subsequent step is determined based on the predicted value of the one or more success metrics, the method provides a physician with objective guidance on which of the candidate intravascular procedure steps to take. This, in turn, helps to improve the success of the procedure, and also to provide more predictable procedural outcomes.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a method of recommending a subsequent step in the performance of an intravascular procedure on a vessel, in accordance with some aspects of the present disclosure
Fig. 2 is a schematic diagram illustrating an example of a system 200 for recommending a subsequent step in the performance of an intravascular procedure on a vessel, in accordance with some aspects of the present disclosure.
Fig. 3 is a schematic diagram illustrating a first example of the operation of calculating of a plaque characteristic map 130 for a vessel from volumetric image data 180, in accordance with some aspects of the present disclosure.
Fig. 4 is a schematic diagram illustrating a second example of the operation of calculating of a plaque characteristic map 130 for a vessel from volumetric image data 180, in accordance with some aspects of the present disclosure.
Fig. 5 illustrates an example of a ground truth plaque characteristic map 130_{GT} representing material properties of the plaque: A) as a cross section perpendicularly through the vessel axis, and B) as a cross section parallel to the vessel axis, in accordance with some aspects of the present disclosure.
Fig. 6 is a schematic diagram illustrating an example of a neural network 150 for predicting a value of a success metric 160_{1..n} obtained by performing one or more candidate intravascular procedure steps on the vessel with one or more candidate intravascular devices, in accordance with some aspects of the present disclosure.
Fig. 7 is a schematic diagram illustrating a second example of a neural network 150 for predicting a value of a success metric 160_{1..n} obtained by performing one or more candidate intravascular procedure steps on the vessel with one or more candidate intravascular devices, and a value of a risk metric, p, representing the presence of vulnerable plaque in the vessel, in accordance with some aspects of the present disclosure.
Fig. 8 is a schematic diagram illustrating an example of a neural network 150 for predicting a value of a success metric 160_{1..n} obtained by performing one or more candidate intravascular procedure steps on the vessel with one or more candidate intravascular devices, and a map of a risk metric 190 representing the presence of vulnerable plaque in the vessel, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to computer-implemented methods that involve of recommending a subsequent step in the performance of an intravascular procedure on a vessel. It is to be appreciated that the methods described herein may be used to recommend a subsequent step in various types of intravascular procedures, including for example angioplasty procedures that are used to widen an obstructed vessel, and mechanical thrombectomy procedures that are used to mechanically remove a thrombus from the vessel. It is also to be appreciated that the methods described herein may be used in intravascular procedures that are performed in various parts of the anatomy, including for example in the coronary vasculature, the peripheral vasculature, the aortic vasculature, and in the carotid artery. Thus, the intravascular procedure may be performed in a vessel, i.e. in vein, or in an artery, within the anatomy in general.

In some examples, reference is made herein to an intravascular device that is used in a mechanical thrombectomy intravascular procedure. At present, there are two main groups of mechanical thrombectomy devices that are used in such procedures: aspiration catheters, and stent retrievers. Examples are described herein wherein the mechanical thrombectomy device is one of these two types of device. However, it is to be appreciated that the computer-implemented methods described herein may also be used with other types of mechanical thrombectomy devices, including coil retrievers, for example. The methods may also be used with other types of intravascular devices, including, for example, ultrasound thrombolysis devices wherein ultrasound energy is used to break-up a thrombus, and rheolytic or rotational embolectomy devices wherein pressurized saline or a rotating device, respectively, are used to macerate or fragment the thrombus which is then aspirated by an aspiration catheter. More generally, it is to be appreciated that the computer-implemented methods described herein may be used to predict intravascular procedural steps in intravascular procedures in general, and that the methods are not limited to use with a particular type of intravascular procedure or device.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above, various imaging techniques are known for determining the pathological, morphological, biomechanical, and material properties of a plaque in a vessel. The properties of the plaque are typically described by plaque characteristic maps, i.e. spatial distributions of these properties. Various techniques are also known for assessing these plaque characteristic maps, and in determining whether the plaque represented in the plaque characteristic maps is "high risk", or in other words "vulnerable", i.e. that it has a high likelihood of causing thrombotic complications, such as myocardial infarction or stroke. Physicians use such risk information in order to decide on the course of treatment of plaque in a vessel. For example, physicians use this information to decide on the immediacy with which to treat a vessel, or which procedural steps to take in the procedure. Physicians also use this information do decide which type of treatment device to use. For example, the physician may use this information to decide which type of stent to use, or whether to extract the thrombus using a stent retriever device, or a direct aspiration device. These choices can have a significant impact on the outcome of the procedure. The risk information helps a physician to determine the course of treatment. However, many of the procedural steps are chosen based to the experience of the physician, which results in variable procedure outcomes.

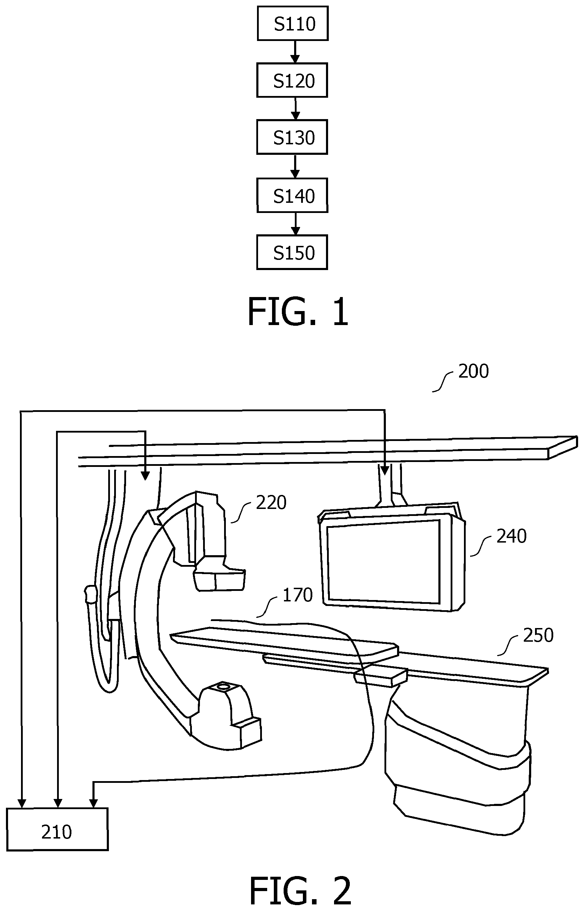

Fig. 1 is a flowchart illustrating an example of a method of recommending a subsequent step in the performance of an intravascular procedure on a vessel, in accordance with some aspects of the present disclosure. With reference to Fig. 1, the method includes:
receiving S 110 one or more plaque characteristic maps 130 for the vessel 120;
receiving S120 2D angiographic data 140 comprising a temporal sequence of images representing the vessel 120 during the intravascular procedure;
inputting S130 the one or more plaque characteristic maps 130, and the 2D angiographic data 140, into a neural network 150;
predicting S140, using the neural network 150, a value of a success metric 160_{1..n} obtained by performing one or more candidate intravascular procedure steps 110_{1..n} on the vessel 120 with one or more candidate intravascular devices 170_{l..j}; and
outputting S150 one of the candidate intravascular procedure steps 110_{1..n} as the recommended subsequent step 110_{k⊂l..n} based on the predicted value of the one or more success metrics 160_{l..n}.

Since, in the above method, the outputted recommended subsequent step is determined based on the predicted value of the one or more success metrics, the method provides a physician with objective guidance on which of the candidate intravascular procedure steps to take. This, in turn, helps to improve the success of the procedure, and also to provide more predictable procedural outcomes.

The method described above may also be implemented by the system illustrated in Fig. 2, which is a schematic diagram illustrating an example of a system 200 for recommending a subsequent step in the performance of an intravascular procedure on a vessel, in accordance with some aspects of the present disclosure. Operations described in relation to the flowchart illustrated in Fig. 1, may therefore also be performed by the system 200 illustrated in Fig. 2, and vice versa.

With reference to the flowchart illustrated in Fig. 1, in the operation S110, one or more plaque characteristic maps 130 for the vessel 120, are received.

The one or more plaque characteristic maps 130 may be received by the one or more processors 210 illustrated in Fig. 2. The one or more plaque characteristic maps 130 may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

A plaque characteristic map 130 represents a spatial distribution of one or more of the following properties of a plaque in the vessel 120: pathological, morphological, biomechanical, and material. As mentioned above, a plaque characteristic map may be generated from various types of volumetric image data representing the vessel. For example, a plaque characteristic map may be generated from CT image data, spectral CT image data, CTA image data, MR image data, MRA image data, external ultrasound image data, and IVUS image data.

The plaque characteristic map(s) that are received in the operation S110 may be generated using various techniques. For instance, the techniques disclosed in the document by Conte, E. et. al., cited above may be used to quantify plaque from CT angiography image data. Techniques for automatically characterizing plaque using pathologic and morphological properties of the plaque extracted from computed tomography angiography "CTA", or magnetic resonance "MR" images, and neural networks, are disclosed in the documents cited above by Hampe, N. et. al., and by Cano-Espinosa, C. et. al., and by Zreik, M. et. al., and by Motwani, M. et. al. The technique disclosed in the document cited above by Sadat, U. et. al., may be used to extract biomechanical information from MR images.

In some examples, the plaque characteristic map(s) are generated using an extra-corporeal imaging system. The use of an extra-corporeal imaging system simplifies workflow by obviating the need to perform an invasive procedure on a subject. In some examples, the plaque characteristic map(s) are generated from pre-procedural volumetric image data. The use of pre-procedural volumetric image data permits the plaque characteristic map(s) to be generated, and also validated, in advance of an intravascular procedure.

Thus, in general, the plaque characteristic map(s) may be generated using volumetric image data representing the vessel, and in some examples, the method described with reference to Fig. 1 also includes:
receiving volumetric image data 180 representing the vessel 120; and
calculating the one or more plaque characteristic maps 130 for the vessel 120 from the volumetric image data 180; and
wherein the received one or more plaque characteristic maps for the vessel are provided by the calculated one or more plaque characteristic maps.

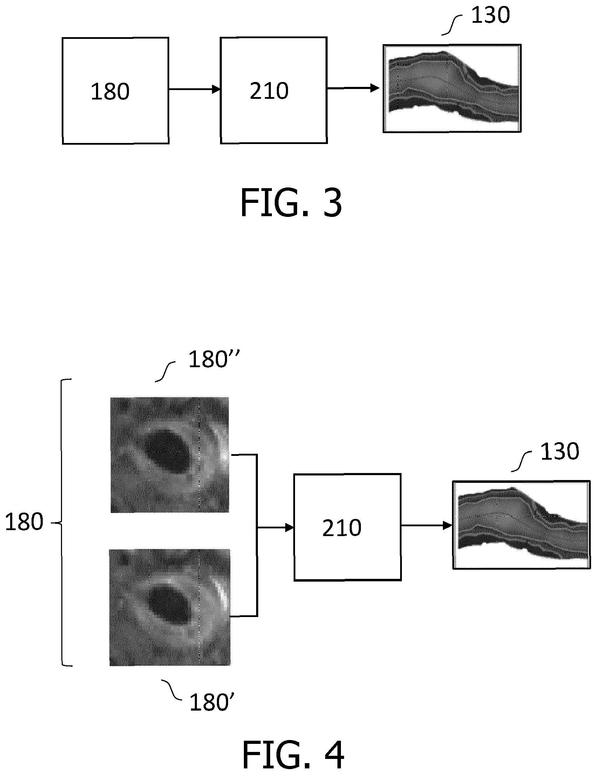

Fig. 3 is a schematic diagram illustrating a first example of the operation of calculating of a plaque characteristic map 130 for a vessel from volumetric image data 180, in accordance with some aspects of the present disclosure. In Fig. 3, the volumetric image data 180 is inputted into one or more processors 210, and the one or more processors 210 calculate the one or more plaque characteristic maps 130 for the vessel 120 from the volumetric image data 180. The characteristics of the plaque are indicated in the plaque characteristic map 130 in Fig. 3 by way of the image intensity values. The one or more processors 210 may form part of the system 200 illustrated in Fig. 2, or they may alternatively be separate to the system 200. In this example, the one or more processors 210 may use the techniques disclosed in the documents cited above to calculate the plaque characteristic map(s) 130 from the inputted volumetric image data 180.

In one example, the volumetric image data 180 used to calculate the one or more plaque characteristic map(s) 130 for the vessel 120 includes contracted state data representing the vessel in a contracted state 180', and expanded state data representing the vessel in an expanded state 180". In this example, the operation of calculating the one or more plaque characteristic maps 130 for the vessel 120 using the volumetric image data 180, is performed using both the contracted state data and the expanded state data for the vessel. This example is described with reference to Fig. 4, which is a schematic diagram illustrating a second example of the operation of calculating of a plaque characteristic map 130 for a vessel from volumetric image data 180, in accordance with some aspects of the present disclosure. In the example illustrated in Fig. 4, the volumetric image data 180 is CT data, and in the CT data representing the vessel in each of the contracted state 180', and the expanded state 180" is inputted into the one or more processors 210 and used to calculate the plaque characteristic map(s) 130. The technique disclosed in the document by Sadat, U. et. al. may be used to calculate the plaque characteristic map(s) 130 from the inputted volumetric image data 180, for example.

The use of both contracted state data and expanded state data to calculate the plaque characteristic maps in the example described above helps to improve the quality of the resulting plaque characteristic map. For example, the quality of biomechanical plaque characteristic map may be improved because this data provides information on the elasticity of the vessel. The contracted state data and the expanded state data for the vessel may correspond to the diastole phase and the systole phase, respectively. The contracted state data and the expanded state data may be acquired using cardiac gating, for instance. By way of an example, CT angiographic images of the vessel in each of these states may be acquired by gating the acquisition of the CT images in response to an electrocardiogram "ECG" signal. The contracted state data and the expanded state data for the vessel may alternatively be acquired in response to the application, and the removal, of an external pressure on the vessel, respectively. The external pressure may be applied by a device, or by a finger, for example. In the former case, the external pressure may be applied by an ultrasound transducer executing a so-called acoustic radiation force impulse "ARFI" technique in which acoustic pulses emitted by the ultrasound transducer induce tissue deformations that are measured using an ultrasound imaging transducer.

A neural network may also be trained to generate the plaque characteristic maps that are received in the operation S110. Thus, in one example, the one or more plaque characteristic maps 130 are calculated by inputting the volumetric image data 180 into a neural network 150; and generating the one or more plaque characteristic maps from the volumetric image data using the neural network.

In this example, inference may be performed with the trained neural network 150 using the one or more processors 210 described with reference to Fig. 3 and Fig. 4. The neural network 150 may be trained to generate a plaque characteristic map 130 for a vessel from the volumetric image data, by:
receiving training data, including a plurality of volumetric training images representing a vessel;
receiving ground truth data, the ground truth data comprising for each of the volumetric training images, a ground truth plaque characteristic map 130_{GT} representing a spatial distribution of one or more of the following properties of a plaque in the vessel: pathological, morphological, biomechanical, and material;
inputting the volumetric training images into the neural network 150; and
for each volumetric training image;
generating a plaque characteristic map for the vessel using the neural network 150; and
adjusting parameters of the neural network 150 based on a difference between the generated plaque characteristic map, and the ground truth characteristic map; and
repeating the inputting, the generating, and the adjusting, until a stopping criterion is met.

In this example, the volumetric training images may be provided by any of the imaging techniques types described above for generating the plaque characteristic maps. In other words, the volumetric training images may include CT data, spectral CT data, CTA data, MR data, MRA data, external ultrasound data, or IVUS data, for example. The corresponding ground truth plaque characteristic maps 130_{GT} may be provided by manually- or automatically-labelled IVUS, optical coherence tomography "OCT", or external 3D ultrasound images that correspond to the volumetric training images. IVUS images that include labels for various tissue types are sometimes referred-to as virtual histology images.

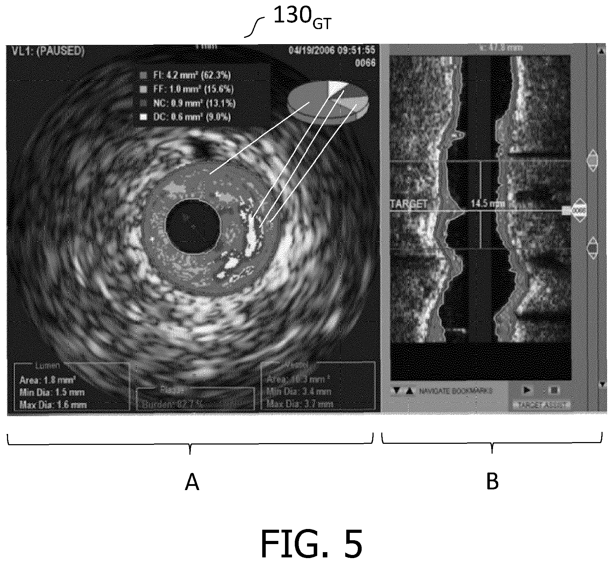

An example of a virtual histology image that is provided by an IVUS image is illustrated in Fig. 5, which illustrates an example of a ground truth plaque characteristic map 130_{GT} representing material properties of the plaque: A) as a cross section perpendicularly through the vessel axis, and B) as a cross section parallel to the vessel axis, in accordance with some aspects of the present disclosure. In Fig. 5, the segmented regions of the images correspond to different material types, as indicated in the pie-chart. The virtual histology images illustrated in Fig. 5 are generated by performing a spectral analysis of the backscattered ultrasound signals detected by an IVUS imaging transducer. Various techniques are known for performing this spectral analysis, such as described in a document by Nair, A. et al. "Automated coronary plaque characterization with intravascular ultrasound backscatter: ex vivo validation", EuroIntervention, 2007 May; 3(1): 113-20. The virtual histology images generated by such techniques permits a distinction between materials such as fibrous tissue, fibro-fatty tissue, necrotic core, and dense calcium, for example.

During training, the ground truth plaque characteristic maps 130_{GT} may also be registered to the corresponding volumetric training images in order to ensure that the neural network learns the correct associations. Thus, in one example, the ground truth plaque characteristic maps 130_{GT} may be registered to the corresponding volumetric training images prior to determining a difference between the generated plaque characteristic map, and the ground truth plaque characteristic map 130_{GT} in the operation of adjusting parameters of the neural network 150. The registration may be performed using known image registration techniques. If the ground truth data is provided by IVUS or OCT image data, the registration may also be performed based on the detection of a position of the IVUS or OCT imaging transducer in the vasculature during the acquisition of image data by the IVUS or OCT imaging transducer. The position of the IVUS or OCT imaging transducer may be detected by performing X-ray projection imaging during pullback of the transducer, determining the position of the transducer in the X-ray images, and registering the X-ray projection images to the volumetric images. This provides a registration between the IVUS or OCT imaging data and the volumetric image data.

In addition to generating plaque characteristic map(s) for the vessel, the neural network 150 may also be trained to predict a value of a risk metric, *p,* representing the presence of vulnerable plaque in the vessel. During inference, the neural network 150 may output the value of the risk metric in order to improve the guidance given to a physician during the interventional procedure. Having this additional risk information may for instance guide the physician's attention to a particular high-risk plaque, or guide the physician to performing the procedure more efficiently in a low-risk vessel.

The network 150 may be trained to predict the value of the risk metric, *p,* by including in the ground truth data, a ground truth value of a risk metric representing the presence of vulnerable plaque in the one or more plaque characteristic maps. The ground truth value may be provided by expert assessment. During training, the parameters of the neural network are adjusted based further on a difference between the predicted values for the risk metric and the corresponding ground truth value. In other words, the neural network 150 described above may also be trained to predict a value of a risk metric, *p,* representing the presence of vulnerable plaque in the vessel by including in the ground truth data, for each of the plurality of vessels:
a ground truth value of a risk metric representing the presence of vulnerable plaque in the one or more plaque characteristic maps; and
wherein the predicting, further comprises predicting, using the neural network, a value of the risk metric representing the presence of vulnerable plaque in the vessel, and
wherein the adjusting parameters of the neural network is based further on a difference between the predicted values for the risk metric and the corresponding ground truth value.

A confidence value associated with the predicted value of the risk metric may also be generated and outputted by the neural network 150 during inference. The confidence value represents a confidence of the predictions made by the neural network 150, and permits decisions to be made based on the risk metric. For example, if the confidence is low, it might be decided not to rely upon the predicted risk metric. A warning may be outputted if the confidence value is below a predetermined threshold value. By way of an example, the neural network 150 may generate the confidence value using the dropout technique. The dropout technique involves iteratively inputting the same data into a neural network and determining the neural network's output whilst randomly excluding a proportion of the neurons from the neural network in each iteration. The outputs of the neural network are then analyzed to provide mean and variance values. The mean value represents the final output, and the magnitude of the variance indicates whether the neural network is consistent in its predictions, in which case the variance is small and the confidence value is relatively higher, or whether the neural network was inconsistent in its predictions, in which case the variance is larger and the confidence value is relatively lower. The neural network 150 may alternatively estimate the confidence value based on an error between the predicted values for the risk metric and the corresponding ground truth value during training. This allows the neural network to learn associations between image features such as quality of images or quality of characteristic maps and a confidence in the predicted value of the risk metric during training. In this way, the trained neural network can generate higher confidence when images of high quality are inputted into the neural network 150 during inference, and lower confidence when images of lower quality are inputted into the neural network 150 during inference.

In general, the neural network 150 that calculates the plaque characteristic maps may include one or more architectures, such as for example a convolutional neural network "CNN", an encoder-decoder network, or one its variants (e.g., variational autoencoder "VAE", maximum mean discrepancy "MMD" VAE, etc.), or a recurrent neural network "RNN" or transformer architecture. The encoder and decoder components of the encoder-decoder network may include a convolutional neural network "CNN" architecture, or a recurrent neural network "RNN" or transformer architecture.

In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network 150 described above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

Returning to the flowchart illustrated in Fig. 1, in the operation S120, 2D angiographic data 140 comprising a temporal sequence of images representing the vessel 120 during the intravascular procedure; is received.

In general, the 2D angiographic data 140 may be generated by a projection X-ray imaging system. Projection X-ray imaging systems typically include a support arm such as a so-called "C-arm", or an "O-arm", that supports an X-ray source-detector arrangement. Projection X-ray imaging systems may alternatively include a support arm with a different shape to these examples. Projection X-ray imaging systems typically generate projection X-ray images with the support arm held in a static position with respect to an imaging region during the acquisition of image data. The temporal sequence of images may be fluoroscopic, i.e. live images. In some examples, the 2D angiographic data 140 may be generated using a digital subtraction angiography "DSA" technique, and wherein each image is generated by subtracting from the image the corresponding pixel intensities of a background image. The 2D angiographic images may be generated by the projection X-ray imaging system 220 illustrated in Fig. 2, for example. By way of an example, the 2D angiographic data 140 may be generated by the Philips Azurion 7 X-ray imaging system marketed by Philips Healthcare, Best, The Netherlands.

The 2D angiographic data 140 that is received in the operation S120 may be received from various sources, including from a projection X-ray imaging system, from a computer readable storage medium, or from the Internet or the Cloud, for example. The 2D angiographic data 140 may be received by the one or more processors 210 illustrated in Fig. 2. The 2D angiographic data 140 may be received via any form of data communication, as described above for the one or more plaque characteristic maps 130.

With continued reference to the flowchart illustrated in Fig. 1, in the operation S130, the one or more plaque characteristic maps 130, and the 2D angiographic data 140, are inputted into a neural network 150. In the operation S 140, the neural network 150 then predicts a value of a success metric 160_{l..n} obtained by performing one or more candidate intravascular procedure steps 110_{l..n} on the vessel 120 with one or more candidate intravascular devices 170_{l..j}. The neural network is trained to predict the value of the success metric 160_{l..n}, as described in more detail below.

In general, the neural network that predicts the value of the success metric 160_{l..n}, and the neural network 150 described above that generates the plaque characteristic maps 130, may form part of the same neural network, or they may be provided by separate neural networks. The neural network that predicts the value of the success metric 160_{l..n} may also be provided by one or more neural networks, as detailed below. In general, the neural network(s) that predict the value of the success metric 160_{l..n} may have a similar architecture to the neural networks described above, and may also be trained using similar techniques.

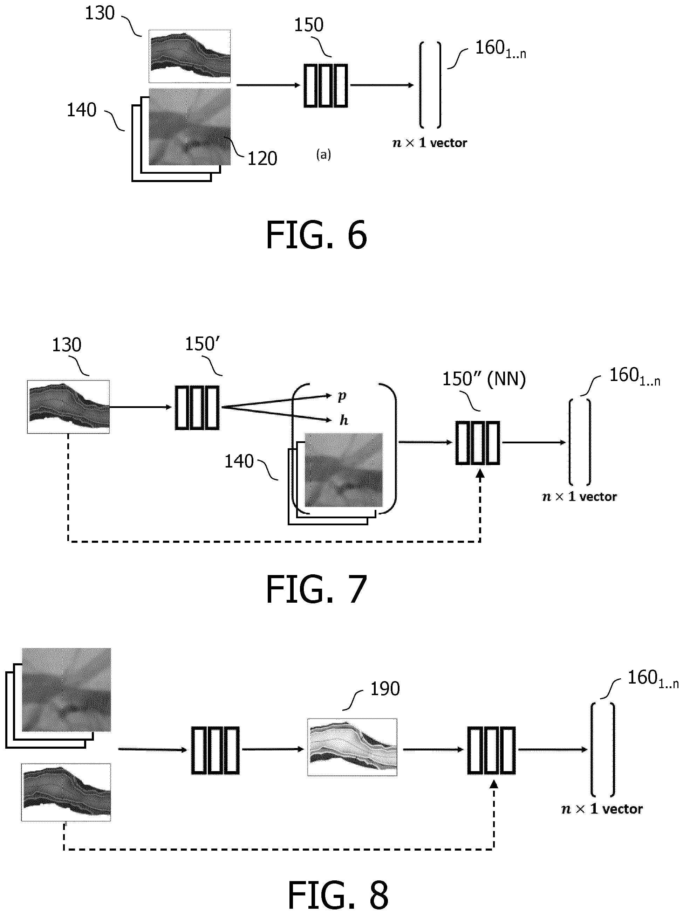

Fig. 6 is a schematic diagram illustrating an example of a neural network 150 for predicting a value of a success metric 160_{l..n} obtained by performing one or more candidate intravascular procedure steps on the vessel with one or more candidate intravascular devices, in accordance with some aspects of the present disclosure. In Fig. 6, the one or more plaque characteristic maps 130, and the 2D angiographic data 140, are inputted into a neural network 150, and the neural network 150 outputs a value of a success metric 160_{l..n} obtained by performing one or more candidate intravascular procedure steps 110_{l..n} on the vessel 120 with one or more candidate intravascular devices 170_{l..j}. Examples of the candidate intravascular procedure steps 110_{l..n} that may be predicted in the operation S 140 include steps such as "perform DSA imaging from (a specified) perspective", "perform balloon angioplasty", "deploy stent", "perform a mechanical thrombectomy procedure", and "wait (a specified number of) minutes before extracting thrombus with stent retriever". The candidate intravascular devices 170_{l..j} to which the value of a success metric 160_{l..n} relates, may be implicit from the type of procedure. For example, it may be implicit from the candidate intravascular procedure step 110_{l..n} "perform balloon angioplasty" that the related candidate intravascular devices 170_{l..j} is a balloon catheter. Alternatively, the related candidate intravascular device 170_{l..j} may be a specific type of candidate intravascular device 170_{l..j}, such as a particular model number, or a device made by a specific manufacturer, or a particular size of the device. Examples of the candidate intravascular devices 170_{l..j} that may be predicted in the operation S140 include a balloon catheter, a stent, a mechanical thrombectomy device, a stent retriever device, a direct aspiration device, a coil retriever, an ultrasound thrombolysis device, and a rheolytic or rotational embolectomy device, for example.

The neural network 150 may be trained to predict a value of a success metric 160_{l..n} obtained by performing one or more candidate intravascular procedure steps 110_{l..n} on the vessel 120 with one or more candidate intravascular devices 170_{l..j}, by:
receiving training data comprising, for each of a plurality of vessels:
   one or more plaque characteristic maps; and
   one or more 2D angiographic images corresponding to the vessel;
receiving ground truth data comprising, for each of the plurality of vessels:
   an indication of a type of an intravascular device employed in a current intravascular procedure step on the vessel; and
   a ground truth value of a success metric obtained by performing the current intravascular procedure step on the vessel with the intravascular device;
   inputting the training data into the neural network 150, and for each of a plurality of vessels in the training data:
      predicting, using the neural network 150, a value of a success metric obtained by performing the current intravascular procedure step on the vessel with the intravascular device; and
      adjusting parameters of the neural network 150 based on a difference between the predicted value for the success metric, and the corresponding ground truth value; and
      repeating the inputting, the predicting, and the adjusting, until a stopping criterion is met.

The training data, and the ground truth data that are used to train the neural network 150 to predict the value of the success metric may in general be curated from records of historic interventional procedures on patient vessels. The vessels represented in the training data may include a range of disease from healthy to atherosclerotic. The training data, and the ground truth data may represent the vessels of multiple patients. Vessels from some tens, hundreds, or even thousands of patients may be used to train the neural network. The training data, and the ground truth data may represent patients having different ages, gender, body mass index, and so forth.

The plaque characteristic maps that are used in the training data may be calculated from volumetric image data for the vessels using the techniques in the documents cited above. Alternatively, the plaque characteristic maps that are used in the training data may be generated by a neural network, as described above with reference to Fig. 3. The plaque characteristic maps represent a spatial distribution of one or more of the following properties of a plaque in each vessel: pathological, morphological, biomechanical, and material. The plaque characteristic maps may be calculated for a specific region of interest in the vessel, such as a diseased region, or a stenosed region, or a healthy region, or a region in which the vessel exhibits a high level of stress. The region of interest may be identified using a bounding box, for example. The region of interest may be identified manually or automatically. For example, a user may define the region of interest manually in the volumetric images for which the plaque characteristic maps are calculated by providing user input via a user input device such as a mouse operating in combination with a graphical user interface "GUI" that displays the volumetric images. Alternatively, the region of interest may be determined automatically using a trained neural network, or a feature detection algorithm, that detects the region of interest in the volumetric images. During training, the neural network may be trained such that it places higher emphasis on data within the region of interest than outside the region of interest, as described in more detail below.

The one or more 2D angiographic images in the training data correspond to the vessel for which the plaque characteristic maps were generated. This data is typically available in records of historic interventional procedures on patient vessels since 2D angiographic imaging such as projection X-ray imaging is often used to guide interventional procedures.

The ground truth data; in particular, an indication of a type of an intravascular device employed in a current intravascular procedure step on the vessel, and a ground truth value of a success metric obtained by performing the current intravascular procedure step on the vessel with the intravascular device; is also typically available in records of historic interventional procedures on patient vessels. The type of intravascular device, e.g. a balloon catheter, used in an intravascular procedure step, e.g. a balloon angioplasty procedure on the vessel, and also the success metric that is obtained, are typically recorded in the notes relating to interventional procedures, and this data may therefore be extracted from the procedure notes so that it can be used in the training data. During training, the type of an intravascular device employed in a historic intravascular procedure step on a vessel may be extracted manually from the procedure notes, and selected by a user from a list of potential devices. Alternatively, the type of intravascular device used in an intravascular procedure step may be determined automatically from the one or more 2D angiographic images that are used in the training data. For example, a feature detection algorithm may be used to detect the type of intravascular device employed in the one or more 2D angiographic images in the training data.

The success metric that is predicted by the neural network, and likewise the ground truth success metric, may in general represent a binary outcome of the step, or a quantitative outcome of the step. An example of a binary outcome is that the intravascular procedure step performed on the vessel with the intravascular device in the training data was either a success or a failure. Examples of quantitative outcomes of the procedure include a probability of the step being successful or unsuccessful, or an amount of re-perfusion achieved by deploying a stent, or a measure of the amount of expansion achieved by deploying a stent, for example. In general, the success metric may represent one or more outcome factors. The outcome factors may for instance include: a speed of the procedure, a measure of completeness of re-perfusion being achieved by the procedure, a 90-day mortality, or a 90-day modified Rankin Scale "mRS", and whether the procedure needed to be repeated. When multiple outcome factors are represented by the success metric, the individual outcome factors may be weighted to provide the success metric. The ground truth value of the success metric that is used to train the neural network 150 may be obtained from the records of historic interventional procedures on patient vessels for which the plaque characteristic maps are calculated.

As mentioned above, the training of the neural network 150 to predict a value of a success metric 160_{l..n} includes the operations of inputting the training data into the neural network 150, predicting the value of a success metric, adjusting parameters of the neural network 150, and repeating the inputting, the predicting, and the adjusting, until a stopping criterion is met. These operations may be carried out by training the neural network in a similar manner to that described above for the neural network that calculates the plaque characteristic maps.

As mentioned above, the neural network may be trained such that it places higher emphasis on data within the region of interest than outside the region of interest. The region of interest may be defined manually or automatically in the training data, i.e. in the 2D angiographic images, or in the plaque characteristic maps. The region of interest may be defined using a GUI, as described above. Training the neural network using such regions of interest helps the neural network to learn specific features. For instance the document by Sadat, U. et al., cited above, mentions that high levels of stress may be important to the calculation of biomechanical maps. Using the region of interest to train the neural network in this manner may therefore result in a more accurate prediction of the success metric. Thus, in one example, the neural network 150 is trained to predict the value of the success metric 160_{l..n}, by further:
receiving input indicative of a region of interest in the vessel in the one or more 2D angiographic images, or in the vessel in the one or more plaque characteristic maps; and
wherein the adjusting parameters of the neural network 150 based on a difference between the predicted values for the success metric, and the corresponding ground truth value; is performed by applying a weighting to the one or more 2D angiographic images, or to the one or more plaque characteristic maps, respectively, and wherein a relatively higher weighting is applied to portions of the one or more 2D angiographic images or to portions of the one or more plaque characteristic maps within the region of interest, respectively, and a relatively lower weighting is applied to portions of the one or more 2D angiographic images or to portions of the one or more plaque characteristic maps outside the region of interest, respectively.

Thus, when training the neural network 150 to predict the value of the success metric 160_{l..n}, the weighting may be applied to the input image(s) directly.

A similar weighting technique may be used when training the neural network 150 to generate a plaque characteristic map 130 for a vessel from volumetric image data. In this case the neural network 150 described with reference to Fig. 3 may be trained to generate a plaque characteristic map for a vessel from the volumetric image data by further:
receiving input indicative of a region of interest in the vessel in the volumetric training images, or in the vessel in the corresponding ground truth plaque characteristic maps 130_{GT}; and
wherein the adjusting parameters of the neural network 150 based on a difference between the generated plaque characteristic map, and the ground truth characteristic map; is performed by applying a weighting to the volumetric training images, or to the ground truth plaque characteristic maps 130_{GT}, or to the difference between the generated plaque characteristic map, and the ground truth characteristic map, respectively, and wherein a relatively higher weighting is applied to portions of the volumetric training images or to portions of the ground truth plaque characteristic maps 130_{GT} or to portions of the difference between the generated and ground truth plaque characteristic maps within the region of interest, respectively, and a relatively lower weighting is applied to portions of the volumetric training images or to portions of the ground truth plaque characteristic maps 130_{GT} or to portions of the difference between the generated and ground truth plaque characteristic maps outside the region of interest, respectively.

In these examples, the input indicative of the region of interest in the vessel may be received by means of a user input device operating in combination with a GUI that displays the 2D angiographic images, the plaque characteristic maps, the volumetric training images, or the ground truth plaque characteristic maps 130_{GT}, as appropriate. Alternatively, the input indicative of the region of interest in the vessel may be automatically inferred from images containing the plaque characteristic maps or automatically computed by a system trained to identify such regions of interest, for instance, a neural network trained to identify plaque in volumetric images.

The neural network 150 that predicts the value of the success metric 160_{l..n} may also predict the value of a risk metric, *p,* representing the presence of vulnerable plaque in the vessel. The neural network described above with reference to Fig. 6 may be trained to predict the value of the risk metric by including ground truth values of the risk metric in the ground truth data. In this example, the ground truth data further comprises, for each of the plurality of vessels, a ground truth value of a risk metric representing the presence of vulnerable plaque in the one or more plaque characteristic maps; and
the predicting the value of the success metric, further comprises predicting, using the neural network, a value of the risk metric representing the presence of vulnerable plaque in the vessel, and
wherein the adjusting parameters of the neural network is based further on a difference between the predicted values for the risk metric and the corresponding ground truth value.

Another neural network that may be used to predict the value of a risk metric is described with reference to Fig. 7, which is a schematic diagram illustrating a second example of a neural network 150 for predicting a value of a success metric 160_{l..n} obtained by performing one or more candidate intravascular procedure steps on the vessel with one or more candidate intravascular devices, and a value of a risk metric, *p,* representing the presence of vulnerable plaque in the vessel, in accordance with some aspects of the present disclosure. As illustrated in Fig. 7, the neural network 150 includes two sections 150' and 150". The plaque characteristic map 130 is inputted into the first section 150' and used to predict the value of a risk metric, *p,* representing the presence of vulnerable plaque in the vessel. The first section 150' of the neural network may be trained to predict the value of the risk metric, *p,* in the manner described above and in which the value of the risk metric, *p,* was predicted by the neural network that generates the plaque characteristic map 130. The confidence values of the risk metric may also be calculated. The risk metric and the confidence values may be outputted. For example, their values may be displayed on a display device, and thereby used to inform a physician of the reliability of the neural network's predictions.

The first section of the neural network 150' illustrated in Fig. 7 also includes a hidden representation of the plaque characteristic map 130, *h,* that is inputted into the second section of the neural network 150" in combination with the 2D angiographic data 140. The second section of the neural network 150" then predicts the success metric 160_{l..n} from the hidden representation, *h*, and from the 2D angiographic data 140. The first and second sections of the neural network, 150', 150", may be trained together, or independently. The value of the risk metric, *p,* may also be inputted into the second section of the neural network 150". The second section of the neural network 150" then predicts the success metric 160_{l..n} from the hidden representation, *h,* and from the value of the risk metric, *p*, and from the 2D angiographic data 140. The plaque characteristic map 130 may additionally be inputted directly into the second section of the neural network 150", as indicated by the dashed line in Fig. 7, whereupon the second section of the neural network 150" uses the plaque characteristic map 130, and the 2D angiographic data 140, to predict the success metric 160_{l..n}.

Fig. 8 is a schematic diagram illustrating an example of a neural network 150 for predicting a value of a success metric 160_{l..n} obtained by performing one or more candidate intravascular procedure steps on the vessel with one or more candidate intravascular devices, and a map of a risk metric 190 representing the presence of vulnerable plaque in the vessel, in accordance with some aspects of the present disclosure. In contrast to the neural networks described with reference to Fig. 6 and Fig. 7, the neural network illustrated in Fig. 8 is trained to generate a 2D or 3D map of the risk metric. By generating this map, spatial information on the risk metric may be provided to a physician, thereby improving the guidance during an interventional procedure. At inference, the 2D angiographic data 140 may be registered to the corresponding plaque characteristic map 130 prior to inputting this data into the neural network.

In this example, the neural network is trained using ground truth data for the 2D or 3D map of the risk metric, as well as ground data representing an indication of a type of an intravascular device employed in a current intravascular procedure step on the vessel; and a ground truth value of a success metric obtained by performing the current intravascular procedure step on the vessel with the intravascular device. The additional ground truth data truth data for the 2D or 3D map of the risk metric may be provided by manually or automatically-annotated IVUS images, or OCT images. Such images may be annotated manually by experts, or annotated automatically using the aforementioned virtual histology techniques. During training, the value of a loss function representing a difference between the ground truth, and predicted 2D or 3D map of the risk metric, is used to adjust parameters of the neural network.

In the Fig. 8 example, the plaque characteristic map 130 may additionally be inputted directly into the second section of the neural network 150", as indicated by the dashed line in Fig. 8, whereupon the second section of the neural network 150" uses the 2D or 3D map of the risk metric, as well as the 2D angiographic data 140, to predict the success metric 160_{l..n}.

Returning to the flowchart illustrated in Fig. 1; in the operation S150, one of the candidate intravascular procedure steps 110_{l..n} are outputted as the recommended subsequent step 110_{k⊂l..n} based on the predicted value of the one or more success metrics 160_{l..n}.

The outputting S150 one of the candidate intravascular procedure steps 110_{l..n} as the recommended subsequent step 110_{k⊂l..n}, may include outputting an identification of the candidate intravascular procedure having the highest value of the success metric 160_{l..n}. In addition to outputting an identification of the candidate intravascular procedure having the highest value of the success metric 160_{l..n}, one or more alternative candidate intravascular procedure steps may also be outputted. For instance, the candidate intravascular procedure steps having the two, or three highest success metrics may be outputted. This allows a physician to decide between alternative steps, in case the steps have similar success metrics, or in case additional factors, such as device availability, time constraints, experience, and so forth need to be taken into account in choosing the next step.

Examples of the candidate intravascular procedure steps 110_{l..n} that may be outputted in the operation S150 include steps such as "perform DSA imaging from (a specified) perspective", "perform balloon angioplasty", "deploy stent", "perform a mechanical thrombectomy procedure", and "wait (a specified number of) minutes before extracting thrombus with stent retriever", for example. The outputting S150 one of the candidate intravascular procedure steps 110_{l..n} may also include outputting an identification of the corresponding candidate intravascular device 170_{l..j}. In some cases, the outputting an identification of the corresponding candidate intravascular device 170_{l..j} may be implicit. For example in the case that the outputted candidate intravascular procedural step 110_{l..n} is "perform balloon angioplasty", it may be implicit that the candidate intravascular device 170_{l..j} is a balloon angioplasty device.

The outputting S150 one of the candidate intravascular procedure steps 1 10_{l..n} may take place in various ways. For instance, the candidate intravascular procedure step may be outputted visually via a display device, such as via the monitor 240 illustrated in Fig. 2, or via a printer, or via an audio output device such as a speaker, or headphones, and so forth. In addition to outputting an identification of the candidate intravascular procedure having the highest value of the success metric 160_{l..n}, the value of the success metric for the candidate intravascular procedure(s) may also be outputted. Outputting the value of the success metric allows a physician to choose between alternative steps that have success metrics with similar values.

The method described with reference to the flowchart illustrated in Fig. 1 may also include one or more additional operations. For example, the plaque characteristic maps may be registered to the 2D angiographic image data and outputted. In this example, the method described with reference to Fig.1 also includes registering the one or more plaque characteristic maps 130 to the 2D angiographic data 140; and
outputting the one or more plaque characteristic maps and the 2D angiographic data as one or more overlay images.

The registration between the plaque characteristic maps and the 2D angiographic image data may be performed using known techniques. The overlay images may be outputted to a computer readable storage medium, or to a display device, such as the monitor 240 illustrated in Fig. 2. Providing this information to a physician may be useful in providing additional context to the calculated success metric, and thereby help the physician to perform the intravascular procedure.

The neural network 150 may also be trained to predict the value of the success metric 160_{l..n} based further on patient data. Patient data such as age, gender, and so forth, may be inputted into the neural network during training, and also during inference, and used to improve the neural network's predictions of the success metric. Such information is typically available from the health records of patients who have undergone past procedures, and may therefore be curated and used to train the neural network 150. Thus, in one example, the neural network 150 is trained to predict the value of the success metric 160_{l..n} based further on patient data; and the method described with reference to Fig. 1 also includes:
receiving patient data relating to the vessel 120;
inputting the patient data into the neural network 150; and
predicting, using the neural network 150, the value of the success metric 160_{l..n} based further on the inputted patient data; and
wherein the patient data comprises one or more of: a patient age, a patient gender, a patient blood pressure, a patient weight, a patient cardiac assessment, and patient smoking history.

In another example, a computer program product, is provided. The computer program product comprises instructions, which when executed by one or more processors, cause the one or more processors to carry out a method of recommending a subsequent step 110_{k⊂l..n} in the performance of an intravascular procedure on a vessel 120. The method comprises:
receiving S 110 one or more plaque characteristic maps 130 for the vessel 120;
receiving S120 2D angiographic data 140 comprising a temporal sequence of images representing the vessel 120 during the intravascular procedure;
inputting S130 the one or more plaque characteristic maps 130, and the 2D angiographic data 140, into a neural network 150;
predicting S140, using the neural network 150, a value of a success metric 160_{l..n} obtained by performing one or more candidate intravascular procedure steps 110_{l..n} on the vessel 120 with one or more candidate intravascular devices 170_{l..j}; and
outputting S150 one of the candidate intravascular procedure steps 110_{l..n} as the recommended subsequent step 1 10_{k⊂l..n} based on the predicted value of the one or more success metrics 160_{l..n}.

In another example, a system 200 for recommending a subsequent step 110_{k⊂l..n} in the performance of an intravascular procedure on a vessel 120, is provided. The system comprises one or more processors 210 configured to:
receive S110 one or more plaque characteristic maps 130 for the vessel 120;
receive S120 2D angiographic data 140 comprising a temporal sequence of images representing the vessel 120 during the intravascular procedure;
input S130 the one or more plaque characteristic maps 130, and the 2D angiographic data 140, into a neural network 150;
predict S140, using the neural network 150, a value of a success metric 160_{l..n} obtained by performing one or more candidate intravascular procedure steps 110_{l..n} on the vessel 120 with one or more candidate intravascular devices 170_{l..j}; and
output S150 one of the candidate intravascular procedure steps 110_{l..n} as the recommended subsequent step 110_{k⊂l..n} based on the predicted value of the one or more success metrics 160_{l..n}.

An example of the system 200 is illustrated in Fig. 2. In some examples, the system 200 may also include one or more of: a projection X-ray imaging system 220 for providing the 2D angiographic data 140, as illustrated in Fig. 2; an intravascular device 170 for performing an intravascular procedure on a vessel, as illustrated in Fig. 2; a display device, such as the monitor 240 illustrated in Fig. 2, for displaying the predicted value of the success metric, the plaque characteristic map(s) 130, the 2D angiographic data 140, the GUI, and other outputs generated by the system 200, and so forth; a patient bed 250; and a user input device configured to receive user input relating to the method described with reference to Fig. 1, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of recommending a subsequent step (110_{k⊂l..n}) in the performance of an intravascular procedure on a vessel (120); the method comprising:
receiving (S110) one or more plaque characteristic maps (130) for the vessel (120);
receiving (S120) 2D angiographic data (140) comprising a temporal sequence of images representing the vessel (120) during the intravascular procedure;
inputting (S130) the one or more plaque characteristic maps (130), and the 2D angiographic data (140), into a neural network (150);
predicting (S140), using the neural network (150), a value of a success metric (160_{l..n}) obtained by performing one or more candidate intravascular procedure steps (110_{l..n}) on the vessel (120) with one or more candidate intravascular devices (170_{l..j}); and
outputting (S150) one of the candidate intravascular procedure steps (110_{l..n}) as the recommended subsequent step (110_{k⊂l..n}) based on the predicted value of the one or more success metrics (160_{l..n}).

2. The computer-implemented method according to claim 1, wherein the neural network (150) is trained to predict the value of the success metric (160_{l..n}) based further on patient data; and wherein the method further comprises:
receiving patient data relating to the vessel (120);
inputting the patient data into the neural network (150); and
predicting, using the neural network (150), the value of the success metric (160_{l..n}) based further on the inputted patient data; and
wherein the patient data comprises one or more of: a patient age, a patient gender, a patient blood pressure, a patient weight, a patient cardiac assessment, and patient smoking history.

3. The computer-implemented method according to claim 1 or claim 2, further comprising:
receiving volumetric image data (180) representing the vessel (120); and
calculating the one or more plaque characteristic maps (130) for the vessel (120) from the volumetric image data (180); and
wherein the received one or more plaque characteristic maps for the vessel are provided by the calculated one or more plaque characteristic maps.

4. The computer-implemented method according to claim 3, wherein the one or more plaque characteristic maps (130) are calculated by:
inputting the volumetric image data (180) into a neural network (150); and
generating the one or more plaque characteristic maps from the volumetric image data using the neural network.

5. The computer-implemented method according to claim 4, wherein the neural network (150) is trained to generate a plaque characteristic map for a vessel from the volumetric image data, by:
receiving training data, including a plurality of volumetric training images representing a vessel;
receiving ground truth data, the ground truth data comprising for each of the volumetric training images, a ground truth plaque characteristic map (130_{GT}) representing a spatial distribution of one or more of the following properties of a plaque in the vessel: pathological, morphological, biomechanical, and material;
inputting the volumetric training images into the neural network (150); and
for each volumetric training image;
generating a plaque characteristic map for the vessel using the neural network (150); and
adjusting parameters of the neural network (150) based on a difference between the generated plaque characteristic map, and the ground truth characteristic map; and
repeating the inputting, the generating, and the adjusting, until a stopping criterion is met.

6. The computer-implemented method according to claim 5, further comprising registering the ground truth plaque characteristic maps 130_{GT} to the corresponding volumetric training images prior to determining a difference between the generated plaque characteristic map, and the ground truth plaque characteristic map (130_{GT}) in the adjusting parameters of the neural network (150).

7. The computer-implemented method according to any one of claims 3 - 6, wherein the volumetric image data (180) comprises contracted state data representing the vessel in a contracted state (180'), and expanded state data representing the vessel in an expanded state (180"); and
wherein the calculating one or more plaque characteristic maps (130) for the vessel (120) using the volumetric image data (180), is performed using both the contracted state data and the expanded state data for the vessel.

8. The computer-implemented method according to claim 7, wherein the contracted state data and the expanded state data for the vessel correspond to the diastole phase and the systole phase, respectively, and wherein the contracted state data and the expanded state data are acquired using cardiac gating;
or
wherein the contracted state data and the expanded state data for the vessel are acquired in response to the application, and the removal, of an external pressure on the vessel, respectively.

9. The computer-implemented method according to any previous claim, further comprising:
predicting, using the neural network (150), a value of a risk metric (p) representing the presence of vulnerable plaque in the vessel; and
outputting the value of the risk metric.

10. The computer-implemented method according to claim 9, further comprising outputting a confidence value for the risk metric (p).

11. The computer-implemented method according to claim 1, wherein the neural network (150) is trained to predict a value of a success metric (160_{l..n}) obtained by performing one or more candidate intravascular procedure steps (110_{l..n}) on the vessel (120) with one or more candidate intravascular devices (170_{l..j}), by:
receiving training data comprising, for each of a plurality of vessels:
one or more plaque characteristic maps; and
one or more 2D angiographic images corresponding to the vessel;
receiving ground truth data comprising, for each of the plurality of vessels:
an indication of a type of an intravascular device employed in a current intravascular procedure step on the vessel; and
a ground truth value of a success metric obtained by performing the current intravascular procedure step on the vessel with the intravascular device;
inputting the training data into the neural network (150), and for each of a plurality of vessels in the training data:
predicting, using the neural network (150), a value of a success metric obtained by performing the current intravascular procedure step on the vessel with the intravascular device; and
adjusting parameters of the neural network (150) based on a difference between the predicted value for the success metric, and the corresponding ground truth value; and
repeating the inputting, the predicting, and the adjusting, until a stopping criterion is met.

12. The computer-implemented method according to claim 11, wherein the neural network (150) is trained to predict the value of the success metric (160_{l..n}), by further:
receiving input indicative of a region of interest in the vessel in the one or more 2D angiographic images, or in the vessel in the one or more plaque characteristic maps; and
wherein the adjusting parameters of the neural network (150) based on a difference between the predicted values for the success metric, and the corresponding ground truth value; is performed by applying a weighting to the one or more 2D angiographic images, or to the one or more plaque characteristic maps, respectively, and wherein a relatively higher weighting is applied to portions of the one or more 2D angiographic images or to portions of the one or more plaque characteristic maps within the region of interest, respectively, and a relatively lower weighting is applied to portions of the one or more 2D angiographic images or to portions of the one or more plaque characteristic maps outside the region of interest, respectively.

13. The computer-implemented method according to any previous claim, further comprising:
registering the one or more plaque characteristic maps (130) to the 2D angiographic data (140); and
outputting the one or more plaque characteristic maps and the 2D angiographic data as one or more overlay images.

14. The computer-implemented method according to any previous claim, wherein the outputting (S150) one of the candidate intravascular procedure steps (110_{l..n}) as the recommended subsequent step (110_{k⊂l..n}), comprises:
outputting an identification of the candidate intravascular procedure having the highest value of the success metric (160_{l..n}), and optionally outputting an identification of the corresponding candidate intravascular device (170_{l..j}).

15. The computer-implemented method according to any previous claim, wherein the plaque characteristic maps (130) represent a spatial distribution of one or more of the following properties of a plaque in the vessel (120): pathological, morphological, biomechanical, and material.
